(19) **Europäisches Patentamt**
**European Patent Office**
**Office européen des brevets**

(11) **EP 1 688 854 B1**

(12) **EUROPEAN PATENT SPECIFICATION**

(45) Date of publication and mention
of the grant of the patent:
**18.03.2009 Bulletin 2009/12**

(51) Int Cl.:
***G06F 19/00*** *(2006.01)* ***A63B 21/00*** *(2006.01)*
***A63B 24/00*** *(2006.01)*

(21) Application number: **06100409.9**

(22) Date of filing: **16.01.2006**

(54) **Multifunctional trainings device with a detachable interactive simulation manipulator**

Multifunktionales Übungsgerät mit einem abnehmbaren Manipulator zur Ermöglichung von interaktiver Simulation

Dispositif d'exercise multifonctionel avec manipulateur détachable pour permettre des simulations interactives

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.02.2005 CN 200510007282**

(43) Date of publication of application:
**09.08.2006 Bulletin 2006/32**

(73) Proprietor: **Lai, Yin-Liang**
**236 Tucheng City (TW)**

(72) Inventor: **Chiang, Johnson**
**236, Tucheng City (CN)**

(74) Representative: **Beck, Michael Rudolf et al**
**Beck & Rössig**
**European Patent Attorneys**
**Cuvilliésstrasse 14**
**81679 München (DE)**

(56) References cited:
**CA-A1- 2 291 058    GB-A- 2 362 331**
**US-A- 5 888 172    US-A- 6 142 913**
**US-A1- 2004 097 331    US-B1- 6 415 188**
**US-B1- 6 530 864**

## Description

**BACKGROUND OF THE PRESENT INVENTION**

**1. Field of the Present Invention**

The invention relates to a multifunctional virtual-reality fitness equipment capably perform an internet 3-dimensional virtual simulation function by using a detachable interactive manipulator as an interface between a fitness equipment and personal computer.

**2. Description of Prior Art**

Due to industrialization, modern people are actually less and less time and short of space to do outdoor sports including jogging and cycling now. However, for solving the problem of large space needed for doing outdoor sports, many indoor fitness equipments, such as a treadmill, exercise bike and rowing machine etc., have rapidly emerged in response to the trends of doing highly effective exercise needed by modern people.

Take a treadmill as an example, different types of treadmill have been developed, invented and popularly used by the public in recent years. Some treadmills are designed to provide better effect in improving and increasing the exercise of runner's arms and legs, and accompanying with sensing the runner's heart beat during exercise.

And, some treadmills are further designed to provide with the function of simulating road surface condition, by combining virtually simulated scenery along the road with the runner's position and speed being capably controlled and detected on the virtually simulated scenery, the runner have a feeling of in reality personally doing outdoor sports on the scene.

Although modern people have already made use of those conventional indoor fitness equipments to achieve the goal of doing exercise, most conventional indoor fitness equipments are always designed and applied for single user to do exercise only, particularly, can not perform the function of multiple users participating in an internet on-line competition game when multiple users are doing exercise together. Nevertheless, as based on the human nature, the user shall be greatly encouraged if some one else can accompany him/her in doing exercise on a kind of novel fitness equipment with function of being connected to personal computer to execute an on-line internet competition game such as jogging, cycling or rowing.

US 2004/0097331 A1 discloses the features of the preamble of claim 1 and describes means for making a connection between the fitness equipment and a computer or video game apparatus. The prior art connection means requires two manipulators that are mounted on the handle bar of a fitness equipment. These manipulators have wires which at their ends are provided with couplers. The wires run through the handle bar and are pulled out from openings in the handlebar so that the couplers can be plugged into a housing. The housing is mounted on the fitness equipment via a separate holder In the housing there is provided electronic circuitry for receiving signals from the manipulators. Further, the circuitry also receives signals from sensors on the fitness equipment via another wire having a connector. This connector is also plugged into the housing. In order to transfer all signals to a computer or video game machine, a third wire is connected to the housing.

If the fitness equipment is to be exchanged because a user in a fitness studio wants to compete in another type of exercise, the assembly and disassembly of the connection will be rather complicated and a cumbersome procedure. There would also be a risk of breaking a wire. Moreover, it would take much time for a user to connect and disconnect the fitness equipment from the computer.

**SUMMARY OF THE PRESENT INVENTION**

Thus, the technical problem of the invention as claimed is to provide a multifunctional virtual-reality fitness equipment with the function of a 3-dimensional virtual image simulation that allows for a quick and easy exchange of the fitness equipment.

This technical problem is solved by a multifunctional virtual-reality fitness equipment according to amended claim 1.

Advantageous embodiments are laid down in further claims.

In view of the above, the major purpose of this present invention is to invent a kind of multifunctional virtual-reality fitness equipment with an invented detachable interactive manipulator for combining conventional fitness equipment with personal computer to perform an internet 3-dimensional virtual simulation.

Another purpose of this present invention is invented a detachable interactive manipulator used on a kind of multifunctional virtual-reality fitness equipment which may automatically detect computer program message of personal computer, if correct message is detected, conventionally operational function on the fitness equipment is stopped, but an internet 3-dimensional virtual simulation is then performed; and user who is doing exercise can select mode of one user doing exercise alone or multiple users participating in on-line competition just to operate on the detachable interactive manipulator according to instruction(s) shown on display of personal compute; however, if no any computer program is detected, the fitness equipment may still be used as a conventional fitness equipment.

Further purpose of this present invention is to disclose an invented detachably interactive manipulator as a reparable controller for multifunctional virtual-reality fitness equipment of the invention, which employs single-chip microcontroller and USB transmission protocol to communicate with personal computer and the fitness equipment, when the fitness equipment is not connected to personal computer, the fitness equipment can still be used as a conventional fitness equipment; whereas, when the fitness equipment is connected to personal computer through the invented detachable interactive manipulator, the fitness equipment shall be served as a multifunctional virtual-reality fitness equipment to perform an internet 3-dimensional virtual simulation function including to perform internet on-line competition game service function.

## BRIEF DESCRIPTION OF THE DRAWINGS

Fig. 1 is a schematic drawing of an embodiment of multifunctional virtual-reality fitness equipment of the invention applied on exercise bike with an internet 3-dimensional virtual simulation function comprising a stationary exercise bike, a personal computer being on-line communication to other fitness equipments through remote internet server, and an invented detachable interactive manipulator equipped with USB interface used to join together the stationary exercise bike and the personal computer.

Fig. 2 is a schematic drawing of another embodiment of multifunctional virtual-reality fitness equipment of the invention applied on rowing machine with an internet 3-dimensional virtual simulation function same as that of exercise bike shown on Fig. 1.

Fig. 3 is a schematic drawing of further another embodiment of multifunctional virtual-reality fitness equipment of the invention applied on treadmill with an internet 3-dimensional virtual simulation function same as that of exercise bike shown on Fig. 1.

Fig. 4 is a structural drawing for a differential strain sensor used on a fitness equipment of the invention.

Fig. 5 is another structural drawing for a differential strain sensor used on a fitness equipment of the invention.

Fig. 6 is front view of the invented detachable interactive manipulator disclosed in the invention.

Fig. 7 shows the configuration of the detachable interactive manipulator of the invention viewed from the reverse side.

Fig. 8 is a schematic drawing showing the arrangement of connecting pins of the chip ST 72651 used on the detachable interactive manipulator of the invention.

Fig. 9 is a block diagram applied on multifunctional virtual-reality fitness equipment of the invention for showing the working principle of application among the invented detachable interactive manipulator, the fitness equipment and the personal computer.

Fig. 10 is a block diagram to show functional or operational principle of a single-chip microcontroller used on the detachable interactive manipulator of the invention.

Fig. 11 is a communication and working block diagram to show operational principle of the invention applied between multiple clients' PC on-line and server.

Fig. 12 is a speed control driving circuit used on the invention for motor speed control by means of varying the voltage gain.

Fig. 13 is a torque control driving circuit to take torque control for an electric motor used on the invention.

## DETAILED DESCRIPTION OF THE PREFERRED EMBODIMENTS

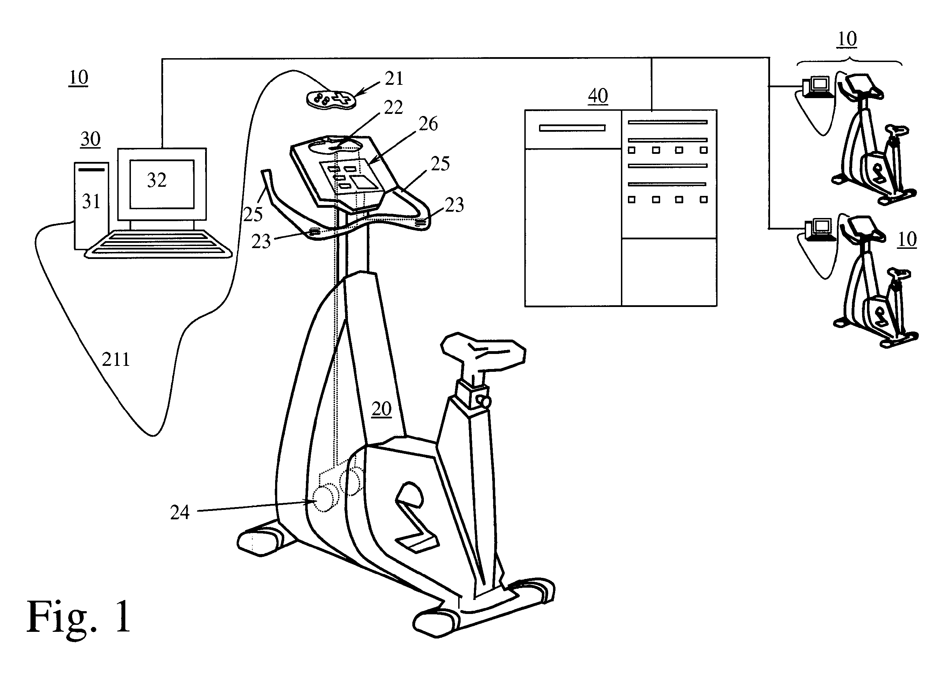

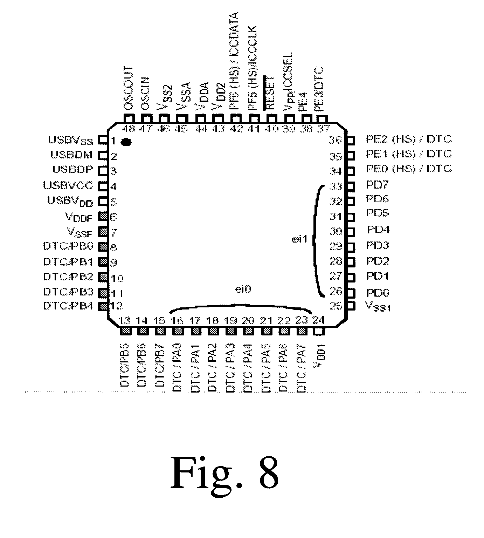

As shown in Figs. 1 to 3, the key feature of this present invention is invented a detachable interactive manipulator 21 served as a separable controller for the invented multifunctional virtual-reality fitness equipment 10. The detachable interactive manipulator 21 is equipped with ST726x series single-chip microcontroller 214 as shown in Fig. 8, so that the detachable interactive manipulator 21 shall be functionally used as a communication and control interface between a personal computer 30 and corresponding fitness equipment 20.

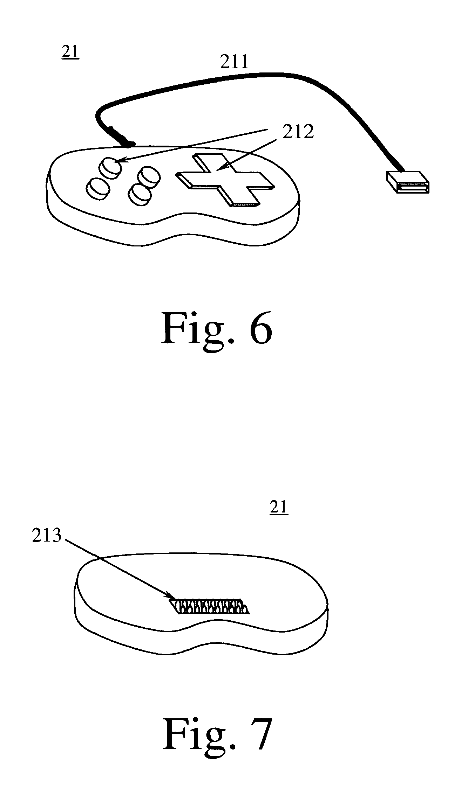

Therefore, as shown in Fig. 6, by only operating pushbuttons 212 arranged on the detachable interactive manipulator 21, user can easily operate the multifunctional virtual-reality fitness equipment 10 of the invention with an internet 3-dimensional virtual simulation function.

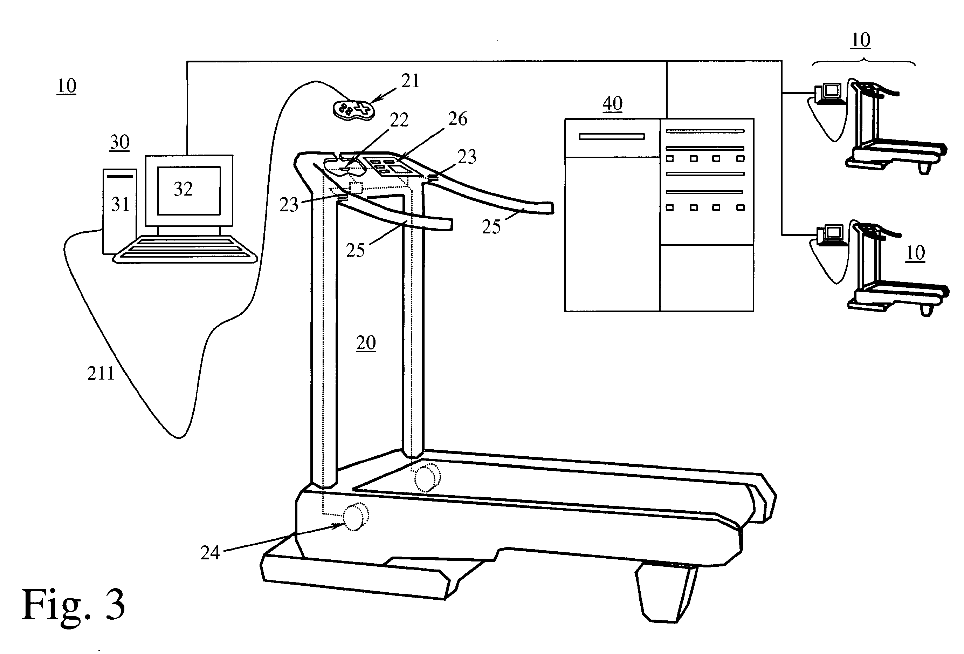

As shown in Fig. 3, to take a treadmill 20 as an illustrated example to the invented multifunctional virtual-reality fitness equipment 10 of the invention, the runner may accord user's interface instructions displayed on the display 32 of personal computer 30 by operating certain pushbutton 212 on the detachable interactive manipulator 21 to select a suitable mode of exercise conditions or requirements to himself or herself.

For example, the runner may select mode of one user doing exercise alone or another mode of multiple users participating in an on-line competition. If mode of one user doing exercise alone is selected, computer processing unit 31 of personal computer 30 will receive a message transmitted from USB transmission line 211 which have been connected to the detachable interactive manipulator 21, and a road scenery image by 3-dimensional virtual simulation is immediately shown on the display 32.

After getting-ready period to start is ended, the runner shall start running exercise according to dynamic image shown on the display 32 and sound effects generated by the personal computer 30. With internet 3-dimensional virtual simulation function, the display 32 may promptly change different road scenery images according to the conditions of running direction and speed of the runner to result in a likely virtual and reality interaction between the runner in doing exercise on the treadmill 20 and the dynamic image shown on the display 32 of personal computer 30.

Since the detachable interactive manipulator 21 of the invention is equipped with ST726x single-chip microcontroller, such as the ST72651 single-chip microcontroller 214 shown in Fig. 8, the detachable interactive manipulator 21 possesses a communication function of USB1.1 with transmission speed of 12 Mbps, and has sixteen sets of digital input, sixteen sets of digital output, eight A/D input ports, and two D/A output ports.

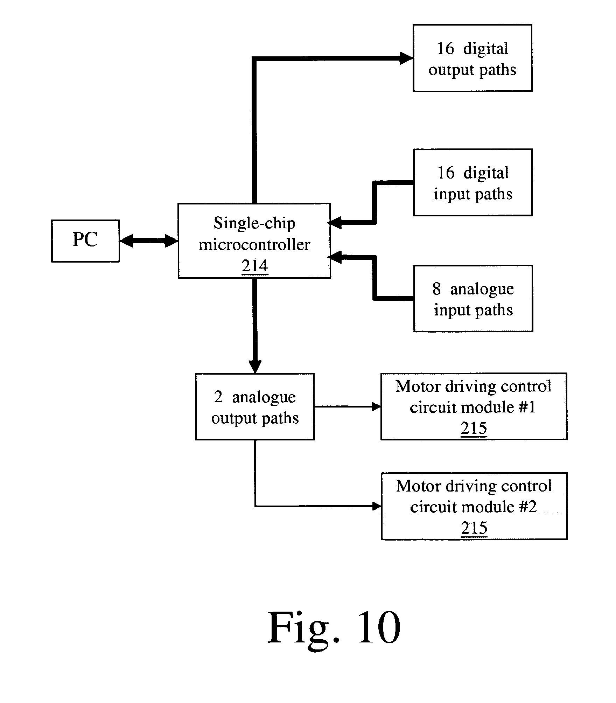

As shown in Fig. 10, the ST72651 single-chip microcontroller 214 of the detachable interactive manipulator 21 shall be installed on and worked with driving control circuit module 215 designed for the invention, and shall be set on software program designed to convert digital command to analogue signal through D/A output ports, so that the detachable interactive manipulator 21 of the invention enable to make torque control or speed control to motors or electric motors installed on the fitness equipment 20.

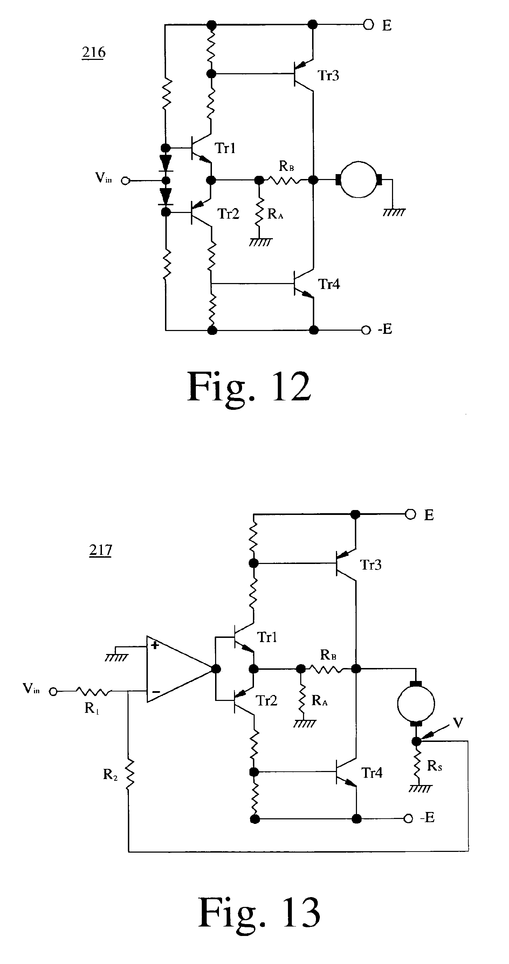

To take speed control, the speed of motors or electric motors installed on the fitness equipment 20 can be controlled merely by varying analogue output voltage through a speed control driving circuit 216 with voltage gain control effect as shown in Fig. 12. If the rated current of the motor or electric motor installed on the fitness equipment 20 is too high, the connection of Tr3 and Tr4 of the speed control driving circuit 216 can be changed to Darlington connection.

To take torque control, a torque control driving circuit 217 as shown in Fig. 13 is used on this present invention, which is a clockwise/counterclockwise rotation driving current control circuit with function of analogue voltage capably varied through the torque control driving circuit 217. A low resistance resistor $R_3$ is connected with motor in series connection for testing and measuring the current. Then, by employing the theory of manipulation amplifier, we have $V_3/R_2 = -Vin/R_1$.

Due to electric current flowing through motor same as that current flowing through Rs, i.e. Ia=Vs/Rs, then Ia = - [R2 / (R1 × Rs)] x Vin.

From the formula it shows that a control of the motor current Ia shall be achieved simply by controlling the voltage Vin, and this will in turn control the output torque of motor.

Further, due to nine sets of digital input of the ST72651 single-chip microcontroller 214 being used to arrange on input pushbuttons 212 of the detachable interactive manipulator 21, the user may operate those input pushbuttons 212 of the detachable interactive manipulator 21 to select a suitable mode of exercise conditions or requirements to himself or herself by following some user's interface instructions shown on the display 32 of personal computer 30.

As shown in Figs. 1, 2, 3 and 10, signals from input pushbutton 212 of the detachable interactive manipulator 21, after decoding by driving control circuit module 215, are transmitted to the computer processing unit 31 of personal computer 30 through USB transmission wire 211 connected between the personal computer 30 and the detachable interactive manipulator 21. Then the computer processing unit 31 will execute some necessary processes and send instruction message to the user through the display 32 of personal computer 30 right after receiving the operating message transmitted from the detachable interactive manipulator 21 to let user know what current state of system is proceeding now and what operation instruction to next step is going to do. Meanwhile, the necessary message or commend is transmitted to the control software hereinafter referred to as "firmware" of ST72651 single-chip microcontroller 214 of the detachable interactive manipulator 21 through USB transmission wire 211. Then the firmware of ST72651 single-chip microcontroller 214 will send necessary output to the hardware according to the message or commend of personal computer 30. The hardware defined in this invention shall include motors controlled by analogue signal, switches or indicating lamps controlled by different digital signal outputs.

When the A/D digital input port of ST72651 single-chip microcontroller 214 of the detachable interactive manipulator 21 is connected to output port of a analogue sensor component of the fitness equipment 20 of the invention, the value, normally the electric potential value, sensed by the analogue sensor component is then converted into digital signal perceivable by computer and transmitted to personal computer 30 through USB transmission wire 211.

Then, after the computer processing unit 31 of personal computer 30 has received those digital signals, the software program set on the computer processing unit 31 of personal computer 30 will immediately identify the meaning of digital values to execute some necessary procedures. In one aspect, the display 32 of personal computer 30 shall simultaneously display the current state of the runner already done including geographical location, speed, direction, pulse, calorie consumption and place etc., in the other aspect, some necessary messages or commends shall through USB transmission wire 211 be output and transmitted to firmware of ST72651 single-chip microcontroller 214 of the detachable interactive manipulator 21. Resulted in the necessary outputs from firmware of the detachable interactive manipulator 21, for corresponding to the messages or commands transmitted from the computer processing unit 31, shall be made to control the fitness equipment 20 of multifunctional virtual-reality fitness equipment 10 of the invention.

As shown in Figs. 1 to 5, the analogue sensor component installed on the fitness equipment 20 of the invented multifunctional virtual-reality fitness equipment 10 includes torque sensor 23 and location sensor 24, wherein the torque sensor 23 may be a set of differential strain sensor which comprises two strain gages mounted on both sides of the handlebar 25 of the fitness equipment 20; in addition, the torque sensor 23 may also be a torque meter. And, the location sensor 24 may be a potentiometer or a location encoder.

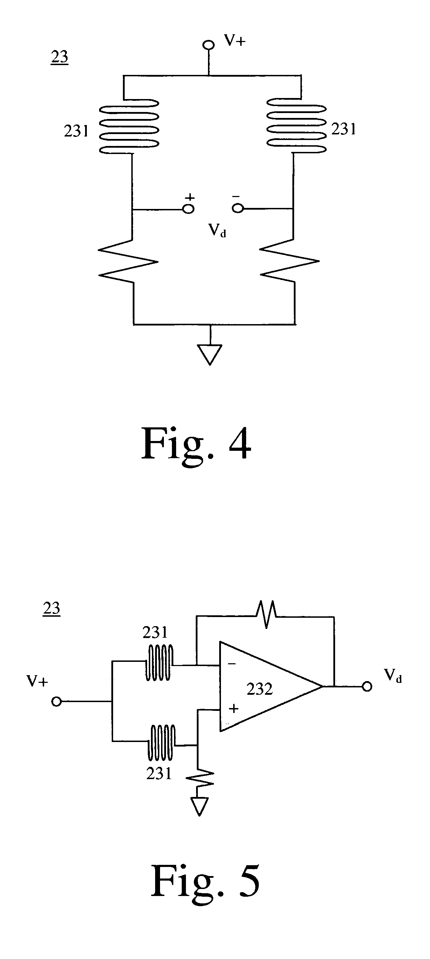

Here are two embodiments of the torque sensor 23 employed by the invention; one of them has structure with strain material 231 shown in Fig. 4. The left and right hand of the user shall exert different torque force on left and right handlebar 25 of the fitness equipment 20 separately that generate different distortion on those strain materials 231 in turn and cause change in resistance value of those strain materials 231 which can be express as follows:

$$\Delta R = R(1+k\frac{(L+\Delta L)^2}{L^2})$$

Where R is the resistance value of the strain material 231 which is not exerted the tensile force; L is the original length of the strain material 231; $\Delta R$ and $\Delta L$ are the increment of the resistance and length of the strain material 231 respectively after an elongation is produced under tensile force; k is the correction factor of stress-strain sensor.

From the above-mentioned formula, it shows that the variation of a resistance depends on the variation of length of the strain material 231, and the variation of length depends on the magnitude of the tensile force exerted on the strain material 231 in axial direction, also the magnitude of tensile force in axial direction depends on the torque force exerted on the handlebar 25 of the fitness equipment 20 by the hand of the user. Therefore, the magnitude of torque exerted by left and right hand of the user will determine the resistance value of the torque sensor 23, this will in turn cause difference in electric potential in left and right side which after conversion calculation according to some specific proportion can represent the magnitude and direction of the angle to turn into by the user during exercise according to the scene image.

The electric potential difference detected by the torque sensor 23 is then converted into computer perceivable digital signal by the A/D port of the detachable interactive manipulator 21 of the invention, and the digital signal is transmitted through USB transmission wire 211 to the software program the computer processing unit 31 of personal computer 30 is executing. Then the software program will immediately identify the turning direction either in left or in right and the turning angle represented by the digital signal, and readjust the scene image according to the required view angle, and finally, the scene image is shown on the display 30 of personal computer 30.

The second type of torque sensor 23 has a structure as shown in Fig. 5 and working principle similar to that of first type of torque sensor 23 mentioned above, different torque force are exerted by the left hand and right hand of the user on the handlebar 25 of the fitness equipment 20 that cause the left and right side strain material 231 to generate different deformation corresponding to the torque exerted by left and right handlebar 25 of the fitness equipment 20. The difference in deformation between left and right strain material 231 of torque sensor 23 will generate an electric potential difference between left side and right side, then the electric potential difference, i.e. the voltage signal, after amplified by amplifier 232 and converted according to some specific proportion, will represent the required direction and angle the user needs to turn. The principle of transmission and conversion of the voltage analogue signal is similar to that of first type of torque sensor 23 mentioned above.

As illustrated in Fig. 1, this multifunctional virtual-reality fitness equipment 10 of the invention is applied on an internet virtual simulation exercise bike 20. The rider on the exercise bike 20 shall determine the direction of the exercise bike 20 in the 3-dimensional virtual simulation scene image simply by exerting different force on left and right handlebar 25, and the amount of displacement of the exercise bike 20 in the 3-dimensional virtual simulation scene image can be determined by the electric potentiometer or location encoder 24 mounted on the pedal of the exercise bike 20.

Take potentiometer as an example, when the pedal of the exercise bike 20 is in different angular position in a cycle the potentiometer will generate different output of different electrical potential difference which can be used for calculating the distance ridden by the rider by way of counting the number of cycles of the pedal traveled.

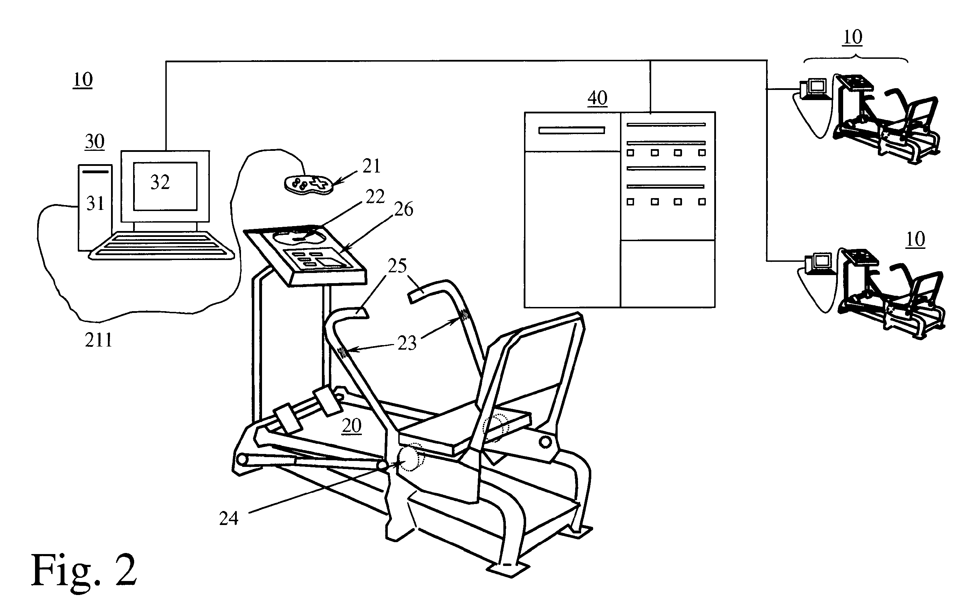

Fig. 2 is another application of this multifunctional virtual-reality fitness equipment 10 of the invention applied on internet virtual simulation sports rowing machine 20. The relative displacement and direction of the rowing machine 20 in 3-dimensional virtual simulation scene image shall be determined in such a way that the analogue signal output of the potentiometer 24 mounted on the oar shaft of the rowing machine 20 is converted into computer perceivable digital signal through the A/D port of the detachable interactive manipulator 21 of the invention, then the digital signal is transmitted through USB transmission wire 211 to the software program of 3-dimensional virtual simulation for rowing which is being executed by the computer processing unit 31 of personal computer 30. The software program set on the computer processing unit 31 will immediately identify these two types of signal, and determine the location and direction of the rowing boat after calculation.

Fig. 3 is further another application of this multifunctional virtual-reality fitness equipment 10 of the invention applied on internet virtual simulation treadmill 20, the direction of runner to run is determined by comparing the force exerted on left and right handlebar 25 of the treadmill 20 by the runner.

From those embodiments as illustrated above, we see that most conventional fitness equipments can be effectively upgraded by combining the fitness equipment with personal computer 31 through the invented detachable interactive manipulator 21 of the invention to form the multifunctional virtual-reality fitness equipment 10 with function of combining internet competition game and 3-dimensional virtual simulation of sports scene image.

The invented detachable interactive manipulator 21 of the invention is shown as in Fig. 6 and 7 which key features is equipped with a ST726x series single-chip microcontroller 214 connected to relative driving control circuit module 215.

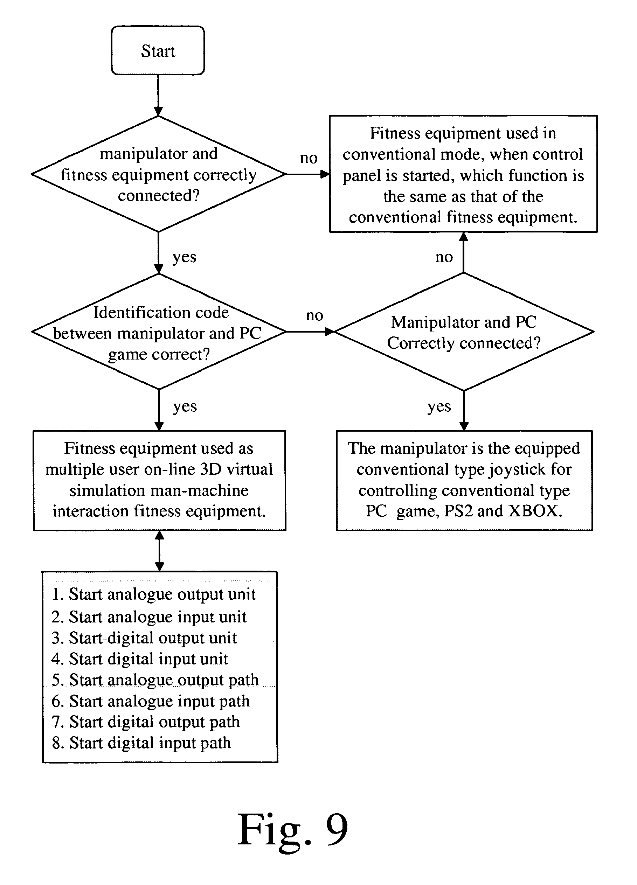

Particularly, the invented detachable interactive manipulator 21 is provided with those working functions depicted as the flow diagram in Fig. 9, when a user is going to use the multifunctional virtual-reality fitness equipment 10 of the invention, the invented detachable interactive manipulator 21 may be selectively joined to or detached from corresponding fitness equipment 20 of the invention by the user.

As shown in Figs. 1 to 3, if the user selects to join the invented detachable interactive manipulator 21 of the invention to the corresponding fitness equipment 20 together, this multifunctional virtual-reality fitness equipment 10 of the invention shall provide function of internet competition game and 3-dimensional virtual simulation of sports scene image to the use.

The invented detachable interactive manipulator 21 has a signal transmission interface such as an USB interface used to connect a signal transmission wire 211, so that the invented detachable interactive manipulator 21 shall be connected to personal computer 30 through signal transmission wire 211, and the user shall input and transmit information to the personal computer 30 through operating the input pushbutton 212 arranged on the face-plate of the invented detachable interactive manipulator 21.

The invented detachable interactive manipulator 21 has 20-pins plug-socket connector 213 arranged on the reverse side as shown in Fig. 7. As shown in Figs. 1 to 3, for constituting a male-to-female connection, each fitness equipment 20 of the invention has a corresponding plug-socket connector 22 arranged on the face-plate 26 of the fitness equipment 20 and designed to enable in connection with 20-pins plug-socket connector 213 of the detachable interactive manipulator 21.

Depending on use's selection, when the 20-pins plug-socket connector 213 of the detachable interactive manipulator 21 and the corresponding plug-socket connector 22 on the fitness equipment 20 are selectively coupled together, each pins of this detachable interactive manipulator 21 of the invention shall be in the following definition:

Pin 0      24 volts power supply;

Pin 1      Analogue signal input 1, used for connecting analogue sensor component output on the fitness equipment 20;

Pin 2      Analogue signal input 2, used for connecting analogue sensor component output on the fitness equipment 20;

Pin 3      Analogue signal input 3, used for connecting analogue sensor component output on the fitness equipment 20;

Pin 4      Analogue signal output 1; used for making torque control or speed control to motors or electric motors on the fitness equipment 20;

Pin 5      Analogue signal output 2; used for making torque control or speed control to motors or electric motors on the fitness equipment 20;

Pin 6      Analogue signal common grounding;

Pin 7      Digital signal output 1, Mode change pin; to take treadmill as an example, when high output, the mode is 3-dimensional virtual simulation treadmill; when low output, the mode is conventional treadmill;

Pin 8      Digital signal output 2, used for controlling digital sensing com input on the fitness equipment 20;

Pin 9      Digital signal output 3, used for controlling digital sensing com input on the fitness equipment 20;

Pin 10     Digital signal output 4, used for controlling digital sensing com input on the fitness equipment 20;

Pin 11     Digital signal output 5, used for controlling digital sensing com input on the fitness equipment 20;

Pin 12     Digital signal input 1, used for connecting digital sensor component output on the fitness equipment 20;

Pin 13     Digital signal input 2, used for connecting digital sensor component output on the fitness equipment 20;

Pin 14     Digital signal input 3, used for connecting digital sensor component output on the fitness equipment 20;

Pin 15     Digital signal input 4, used for connecting digital sensor component output on the fitness equipment 20;

Pin 16     Digital signal input 5, used for connecting digital sensor component output on the fitness equipment 20;

Pin 17     Digital signal input 6, used for connecting digital sensor component output on the fitness equipment 20;

Pin 18     Digital signal input 7, used for connecting digital sensor component output on the fitness equipment 20;

Pin 19     Digital signal common grounding.

Therefore, when a user selects the invented detachable interactive manipulator 21 connected to corresponding fitness equipment 20, the ST726x series single-chip microcontroller 214 of the invented detachable interactive manipulator 21 will possess the function as depicted in Fig. 10 to enable the fitness equipment 20 in interaction with the personal computer 30, the user shall enter on internet competition game with 3-dimensional virtual simulation of sports scene image.

As shown in Fig. 9 and in conjunction with the above-mentioned embodiments, when the detachable interactive manipulator 21 of the invention is connected to personal computer 30, it can communicate with the software being executed by the computer processing unit 31 of personal computer 30 to conform whether the identification code is correct or not. If it is correct, the software set in processing unit 31 of personal computer 30 and the firmware of the detachable interactive manipulator 21 can operate in normal condition, otherwise, the software set in the computer processing unit 31 will have the program retreated, and indicate that the identification code of the detachable interactive manipulator 21 is wrong that results in the failure of achieving interaction between the fitness equipment 20 and the 3-dimensional virtual simulation software.

In case the connection between the detachable interactive manipulator 21 and the personal computer 30 is not successfully established, or, the 3-dimensional virtual simulation software is not started, the detachable interactive manipulator 21 will automatically switch the operating mode to the general operating mode after the detachable interactive manipulator 21 is connected to the fitness equipment 20; while in this case the fitness equipment 20 shall operate in the conventional function, the user may directly operate the equipment with the function shown on control panel 26 of the fitness equipment 20.

If the identification code of the detachable interactive manipulator 21 is examined to be correct, the 3-dimensional virtual simulation software of personal computer 30 shall completely communicate with the fitness equipment 20 through the firmware of the detachable interactive manipulator 21.

When the detachable interactive manipulator 21 and the 3-dimensional virtual simulation software of personal computer 30 are joined together, the detachable interactive manipulator 21 will automatically switch to 3-dimensional virtual simulation mode, and then the fitness equipment 20 will possess the functions including conventional operation, and internet competition game with 3-dimensional virtual simulation of sports scene image.

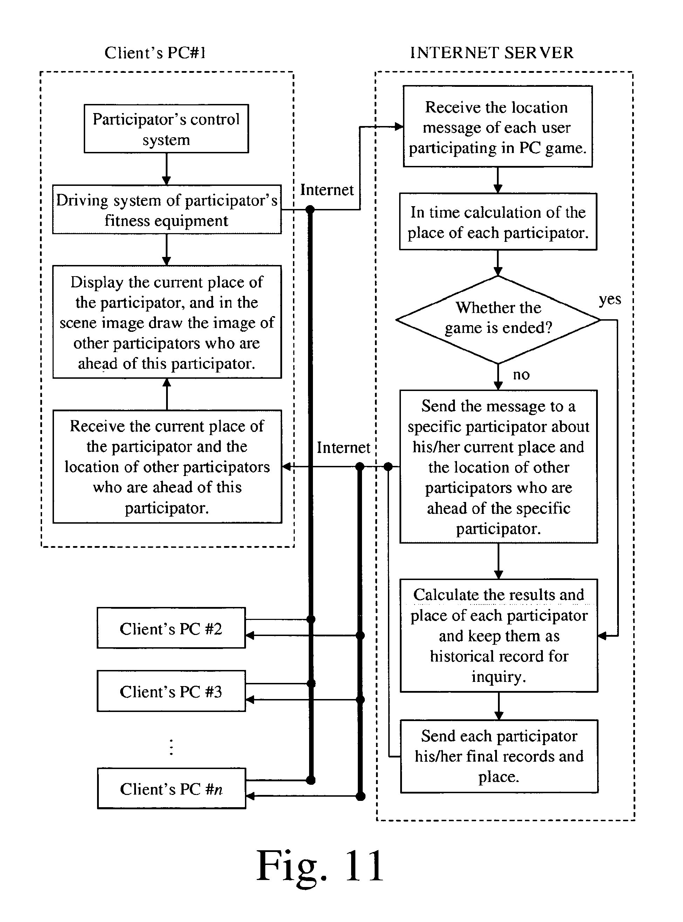

The flow diagram shown in Fig. 11 illustrates the communication and processing work between personal computers 30 of the clients and an internet server 40 during executing the multiple user on-line internet competition game function.

As illustrated in Figs. 1 to 3, this multifunctional virtual-reality fitness equipment 10 of this present invention may be applied on different kinds of conventional fitness equipment, and particularly possess the function of 3-dimensional virtual simulation image for single user doing exercise by himself/herself or for multiple users voluntarily participating in on-line contest with others through internet.

In case of executing internet on-line competition game, the manner to carry out the internet game is shown in Fig. 11. User can operate the input pushbutton 212 on the detachable interactive manipulator 21 to select the desired mode such as one user doing exercise or multiple users carrying on-line sports competition game.

However, if multiple users are carrying out on-line sports competition game, the required message, such as the total number of persons participating the game at present and the time to start count-down etc., shall be updated and sent in time to the personal computer 30 of every participator, then the message shall be displayed on the display 32 of personal computer 30 for the participator to prepare for the game according to the time message shown on the display 32.

After the game starts, the detachable interactive manipulator 21 of each participator will in time receive the analogue or digital signal from torque sensor 23 and location sensor 24. The signal, after being converted into digital type, shall be transmitted to computer processing unit 31 of personal computer 30 through USB transmission wire 211, and read by the 3-dimensional virtual simulation software being executed by the personal computer 30 to determine the relative location, speed and direction of the participator presently in the 3-dimensional virtual simulation scene image, and the message of the relative location of the user shall be sent to internet server 40 for calculating the place of each participator based on the relative location message, and then the message including the place of each participator and the relative location of 5~10 participators who are ahead of a specific participator shall be transmitted back to the computer 30 of the specific participator, and the display 32 of the participator's computer 30 will display the place of himself/herself, and in time display the dynamic virtual image of 5~10 participators who are ahead of himself/herself at the relative location of the 3-dimensional virtual simulation scene image.

Besides the display 32 of personal computer 30 will also display the personal information of the user including current location, speed, direction, pulse, calorie consumption etc., and the record achieved by the user in a competition game will be kept in server 40 as historical record.

In case the user selects one person doing exercise alone, the display 32 of personal computer 30 will perform the same as that in the multiple user participation mode except the information of other participators and the place of the participator in doing exercise.

## Claims

1. A multifunctional virtual-reality fitness equipment (10) with the function of a 3-dimensional virtual image simulation for a single user doing exercise alone or participating in an on-line competition with others through a remote internet server (40), comprising:

   - a fitness equipment (20),
   - an interactive manipulator (21) adapted to be detachably mounted on the fitness equipment (20) and connected to a personal computer (30) that has on-line communication with other fitness equipments (20) through the remote internet server (40), wherein the detachable interactive manipulator (21) is an input/output device for the user,

**characterized in that**

- the detachable interactive manipulator (21) comprises:

a signal transmission interface and a signal transmission wire (211) for connecting said detachable interactive manipulator (21) directly to the personal computer (30),
a plug-socket connector (213) on its reverse side, possessing plural analogue signal input pins and analogue signal output pins, and
a single-chip microcontroller (214) for converting digital information transmitted from the signal transmission wire (211) into analogue information, transmitting analogue signals to the analogue signal output pins of the plug-socket connector (213), converting analogue signals from the analogue signal input pins of the plug-socket connector (213) into digital information, and transmitting such digital information to the personal computer (30);

- the fitness equipment (20) comprises:

a face-plate (26),
a plug-socket connector (22) provided on the face plate (26), said plug-socket connector (22) being connectable to the plug-socket connector (22) of the detachable interactive manipulator (21) and has plural analogue signal input pins corresponding to analogue signal output pins of the plug-socket connector (213) and plural analogue signal output pins corresponding to analogue signal input pins of the plug-socket connector (213), and
one or more than one torque and/or location sensors (23, 24) connected to analogue signal output pins of the plug-socket connector (22) on the face plate (26);

- wherein the detachable interactive manipulator (21) is adapted for transmitting signals from the fitness equipment (20) to the personal computer (30) for simulating a 3-dimensional virtual image scene; and
- wherein the detachable interactive manipulator (21) is adapted to enable a user to select to either join or detach it from the corresponding fitness equipment (20) and when joined to the corresponding fitness equipment (20) to select either doing exercise alone or doing exercise with multiple users participating in an on-line competition.

2. The internet multifunctional virtual-reality fitness equipment as defined in claim 1, **characterized in that** the torque sensor (23) is a set of differential strain sensors comprising two strain gages mounted on both sides of handlebar (25) of the fitness equipment (20).

3. The internet multifunctional virtual-reality fitness equipment as defined in claim 1, **characterized in that** a ST726x series single-chip microcontroller (214) provides function of identifying and determining whether connection in between the detachable interactive manipulator (21) and the fitness equipment (20) is correct or not.

4. The internet multifunctional virtual-reality fitness equipment as defined in claim 1, **characterized in that** a ST726x series single-chip microcontroller (214) further provides function of identification code used for the detachable interactive manipulator (21) and the personal computer (30) to identify each other, if identification code is checked to be correct, 3-dimensional virtual simulation software of the personal computer (30) shall communicate with the fitness equipment (20) through the detachable interactive manipulator (21).

5. The internet multifunctional virtual-reality fitness equipment as defined in one of claims 1 to 4, **characterized in that** the plug-socket connectors (22, 213) are 20-pin connectors.

**Patentansprüche**

1. Multifunktionales Virtual-Reality-Fitnessgerät (10) mit der Funktion einer dreidimensionalen virtuellen Bildsimulation für einen einzelnen Nutzer, der alleine trainiert oder an einem Online-Wettbewerb mit anderen über einen Remote-Internet-Server (40) teilnimmt, umfassend:

- ein Fitnessgerät (20),
- eine interaktive Handhabungsvorrichtung (21), die lösbar an dem Fitnessgerät (20) befestigbar ist und mit einem Personalcomputer (30) verbunden ist, der zu anderen Fitnessgeräten (20) über den Remote-Internet-Server (40) in Onlineverbindung steht, wobei die lösbare interaktive Handhabungsvorrichtung (21) ein Eingabe/ Ausgabegerät für den Benutzer ist,

**dadurch gekennzeichnet, dass**

- die lösbare interaktive Handhabungsvorrichtung (21) umfasst:

eine Signalübertragungsschnittstelle und ein Signalübertragungskabel (211) zur Verbindung der lösbaren interaktiven Handhabungsvorrichtung (21) unmittelbar mit dem Personalcomputer (30),
eine Steckverbindungseinrichtung (213) an der Rückseite, welche eine Vielzahl von analogen Signaleingangspins und analogen Signalausgangspins aufweist, und
einen Einzelchip-Microcontroller (214) zum Konvertieren digitaler Information, welche durch das Signalübertragungskabel (211) übertragen wird, in analoge Information, zum Übertragen analoger Signale zu den analogen Signalausgangspins der Steckverbindungseinrichtung (213), zum Konvertieren analoger Signale der analogen Signaleingangspins der Steckverbindungseinrichtung (213) in digitale Information, und zum Übertragen solcher digitaler Information an den Personalcomputer (30);

- das Fitnessgerät (20) umfasst:

eine Stirnplatte (26),
eine Steckverbindungseinrichtung (22) an der Stirnplatte (26), wobei die Steckverbindungseinrichtung (22) mit der Steckverbindungseinrichtung (213) der lösbaren interaktiven Handhabungsvorrichtung (21) verbindbar ist und eine Vielzahl analoger Signaleingangspins entsprechend den analogen Signalausgangspins der Steckverbindungseinrichtung (213) und eine Vielzahl analoger Signalausgangspins entsprechend der Anzahl der analogen Signaleingangspins der Steckverbindungseinrichtung (213) aufweist, und
einen oder mehr als einen Drehmoment- und/oder Positionssensor (23, 24), die mit den analogen Signaleingangspins der Steckverbindungseinrichtung (22) an der Stirnplatte (26) verbunden sind;

wobei die lösbare interaktive Handhabungsvorrichtung (21) derart ausgebildet ist, um Signale von dem Fitnessgerät (20) zu dem Personalcomputer (30) zu übertragen, um eine dreidimensionale virtuelle Bildszene zu simulieren; und
wobei die lösbare interaktive Handhabungsvorrichtung (21) derart ausgebildet ist, um einem Benutzer zu ermöglichen, diese an dem entsprechenden Fitnessgerät (20) anzubringen oder von diesem zu lösen, und, sofern diese an dem entsprechenden Fitnessgerät (20) angebracht ist, die Wahl zu ermöglichen zwischen einem Trainieren allein oder einem Trainieren zusammen mit einer Vielzahl von Benutzern in einem Online-Wettbewerb.

2. Multifunktionales Internet-Virtual-Reality-Fitnessgerät (10) nach Anspruch 1, **dadurch gekennzeichnet, dass** der Drehmomentsensor (23) aus einer Gruppe von Differenzdehnungssensoren besteht, welche zwei Dehnungsmesseinrichtungen an beiden Seiten einer Handabstützungseinrichtung (25) des Fitnessgeräts (20) aufweist.

3. Multifunktionales Internet-Virtual-Reality-Fitnessgerät (10) nach Anspruch 1, **dadurch gekennzeichnet, dass** ein Einzelchip-Microcontroller (214) vom Typ ST726x die Funktion der Identifikation und Bestimmung der Korrektheit der Verbindung zwischen der lösbaren interaktiven Handhabungsvorrichtung (21) und dem Fitnessgerät (20) bereitstellt.

4. Multifunktionales Internet-Virtual-Reality-Fitnessgerät (10) nach Anspruch 4, **dadurch gekennzeichnet, dass** der Einzelchip-Microcontroller (214) vom Typ ST726x weiterhin eine Identifikationscodefunktion bereitstellt, die dazu verwendet wird, dass die lösbare interaktive Handhabungsvorrichtung (21) und der Personalcomputer (30) einander identifizieren können, wobei, sofern ein korrekter Identifikationscode festgestellt wird, die dreidimensionale virtuelle Simulationssoftware des Personalcomputers (30) mit dem Fitnessgerät (20) über die lösbare interaktive Handhabungsvorrichtung (21) kommuniziert.

5. Multifunktionales Internet-Viitual-Reality-Fitnessgerät (10) nach einem der Ansprüche 1 bis 4, **dadurch gekennzeichnet, dass** die Steckverbindungseinrichtungen (22, 213) 20-Pin-Steckverbindungseinrichtungen sind.

**Revendications**

1. Dispositif d'exercice virtuel multifonctionnel (10) avec la fonction d'une simulation d'image virtuelle tridimensionnelle pour un seul utilisateur faisant de l'exercice seul ou participant à une compétition en ligne avec d'autres par un serveur internet à distance (40) comprenant :

- un dispositif d'exercice (20),
- un manipulateur interactif (21) adapté pour être monté de manière amovible sur le dispositif d'exercice (20) et relié à un ordinateur personnel (30) qui est en communication en ligne avec d'autres dispositifs d'exercice (20) par le serveur internet à distance (40) dans lequel le manipulateur interactif amovible (21) est un dispositif entrée/sortie pour l'utilisateur,

**caractérisé en ce que**

- le manipulateur interactif amovible (21) comprend :

une interface de transmission de signal et un fil de transmission de signal (211) pour relier ledit manipulateur interactif amovible (21) directement à l'ordinateur personnel (30),
un connecteur mâle et femelle (213) sur son côté inverse possédant plusieurs broches d'entrée de signal analogiques et des broches de sortie de signal analogiques et
un micro-contrôleur sur une seule puce (214) pour convertir des informations numériques transmises par le fil de transmission de signal (211) en informations analogiques, transmettre des signaux analogiques aux broches de sortie de signaux analogiques du connecteur mâle et femelle (213), convertir des signaux analogiques des broches d'entrée de signaux analogiques du connecteur mâle et femelle (213) en informations numériques et transmettre ces informations numériques à l'ordinateur personnel (30) ;

- le dispositif d'exercice (20) comprend:

une plaque frontale (26),
un connecteur mâle et femelle (22) prévu sur la plaque frontale (26), ledit connecteur mâle et femelle (22) pouvant être relié au connecteur mâle et femelle (22) du manipulateur interactif amovible (21) et ayant plusieurs broches d'entrée de signaux analogiques correspondant aux broches de sortie de signaux analogiques du connecteur mâle et femelle (213) et plusieurs broches de sortie de signaux analogiques correspondant aux broches d'entrée de signaux analogiques du connecteur mâle et femelle (213) et
un ou plusieurs détecteurs de torsion et/ou de position (23, 24) reliés à des broches de sortie de signaux analogiques du connecteur mâle et femelle (22) sur la plaque frontale (26);

- dans lequel le manipulateur interactif amovible (21) est adapté pour transmettre des signaux du dispositif d'exercice (20) à l'ordinateur personnel (30) pour simuler une scène d'image virtuelle tridimensionnelle et
- dans lequel le manipulateur interactif amovible (21) est adapté pour permettre à l'utilisateur de choisir soit de le relier ou de le détacher du dispositif d'exercice correspondant (20) et, lorsqu'il est relié au dispositif d'exercice correspondant (20), de choisir soit de faire de l'exercice seul, soit de faire de l'exercice avec des utilisateurs multiples qui participent à une compétition en ligne.

2. Dispositif d'exercice virtuel multifonctionnel internet selon la revendication 1, **caractérisé en ce que** le détecteur de torsion (23) est un ensemble de détecteurs d'effort différentiels comprenant deux jauges d'effort montées des deux côtés du guidon du dispositif d'exercice (20).

3. Dispositif d'exercice virtuel multifonctionnel internet selon la revendication 1, **caractérisé en ce qu'**un micro-contrôleur série sur une seule puce ST726x (214) fournit la fonction d'identification et de détermination de si la connexion entre le manipulateur interactif amovible (21) et le dispositif d'exercice (20) est correcte ou non.

4. Dispositif d'exercice virtuel multifonctionnel internet selon la revendication 1, **caractérisé en ce qu'**un micro-contrôleur série sur une seule puce ST726x (214) fournit de plus la fonction de code d'identification utilisé pour le manipulateur interactif amovible (21) et l'ordinateur personnel (30) pour identifier mutuellement, si le code d'identification contrôlé est correct, le logiciel de simulation virtuelle tridimensionnelle de l'ordinateur personnel (30) communiquera avec le dispositif d'exercice (20) par l'intermédiaire du manipulateur interactif amovible (21).

5. Dispositif d'exercice virtuel multifonctionnel internet selon l'une des revendications 1 à 4, **caractérisé en ce que** les connecteurs mâles et femelles (22, 213) sont des connecteurs à 20 broches.

Fig. 1

Fig. 2

10

30

31    32

21

22    26

211

25

23

24

20

40

10

10

EP 1 688 854 B1

12

Fig. 3

EP 1 688 854 B1

23

231

231

V+

+

−

$V_d$

# Fig. 4

23

231

231

V+

−

232

+

$V_d$

231

# Fig. 5

21

211

212

# Fig. 6

21

213

# Fig. 7

Fig. 8

Start

manipulator and fitness equipment correctly connected? — no → Fitness equipment used in conventional mode, when control panel is started, which function is the same as that of the conventional fitness equipment.

yes

Identification code between manipulator and PC game correct? — no → Manipulator and PC Correctly connected?

no

yes

yes

Fitness equipment used as multiple user on-line 3D virtual simulation man-machine interaction fitness equipment.

The manipulator is the equipped conventional type joystick for controlling conventional type PC game, PS2 and XBOX.

1. Start analogue output unit
2. Start analogue input unit
3. Start digital output unit
4. Start digital input unit
5. Start analogue output path
6. Start analogue input path
7. Start digital output path
8. Start digital input path

# Fig. 9

| 16 digital output paths |
| --- |

| 16 digital input paths |
| --- |

| PC | | Single-chip microcontroller 214 |

| 8 analogue input paths |
| --- |

| 2 analogue output paths |
| --- |

| Motor driving control circuit module #1 215 |
| --- |

| Motor driving control circuit module #2 215 |
| --- |

# Fig. 10

Client's PC#1INTERNET SERVER

Participator's control system

Driving system of participator's fitness equipment

Internet

Display the current place of the participator, and in the scene image draw the image of other participators who are ahead of this participator.

Receive the current place of the participator and the location of other participators who are ahead of this participator.

Internet

Receive the location message of each user participating in PC game.

In time calculation of the place of each participator.

Whether the game is ended?  yes

no

Send the message to a specific participator about his/her current place and the location of other participators who are ahead of the specific participator.

Calculate the results and place of each participator and keep them as historical record for inquiry.

Client's PC #2

Client's PC #3

⋮

Client's PC #n

Send each participator his/her final records and place.

# Fig. 11

Fig. 12

Fig. 13

**EP 1 688 854 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040097331 A1